Europäisches Patentamt

European Patent Office

Office européen des brevets

(19)

(11) **EP 1 324 824 B1**

(12) **EUROPÄISCHE PATENTSCHRIFT**

(45) Veröffentlichungstag und Bekanntmachung des Hinweises auf die Patenterteilung:
**02.06.2004 Patentblatt 2004/23**

(51) Int Cl.$^7$: **B01J 23/887**, B01J 37/00, B01J 35/00, C07C 51/25, C07C 57/04

(21) Anmeldenummer: **01972074.7**

(22) Anmeldetag: **20.09.2001**

(86) Internationale Anmeldenummer:
**PCT/EP2001/010910**

(87) Internationale Veröffentlichungsnummer:
**WO 2002/024327 (28.03.2002 Gazette 2002/12)**

(54) **VERFAHREN ZUR HERSTELLUNG VON MEHRPHASIGEN MULTIMETALLOXIDMASSEN**

METHOD FOR PRODUCING MULTIPLE-PHASE MULTI-METAL OXIDE MATERIALS

PROCEDE DE PRODUCTION DE MATIERE A PHASES MULTIPLES A BASE D'OXYDE POLYMETALLIQUE

(84) Benannte Vertragsstaaten:
**DE**

(30) Priorität: **21.09.2000 DE 10046928**

(43) Veröffentlichungstag der Anmeldung:
**09.07.2003 Patentblatt 2003/28**

(73) Patentinhaber: **BASF AKTIENGESELLSCHAFT**
**67056 Ludwigshafen (DE)**

(72) Erfinder:
  • **UNVERRICHT, Signe**
    **68169 Mannheim (DE)**
  • **FELDER, Raimund**
    **67434 Neustadt (DE)**
  • **ARNOLD, Heiko**
    **210014 Nanjing (CN)**

• **PETZOLDT, Jochen**
  **68163 Mannheim (DE)**

(74) Vertreter: **Thalhammer, Wolfgang, Dr. et al**
**Reitstötter, Kinzebach & Partner,**
**Sternwartstrasse 4**
**81679 München (DE)**

(56) Entgegenhaltungen:
**EP-A- 0 061 830        EP-A- 0 756 894**
**DE-A- 19 815 281**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Multimetalloxidmassen, die als aktive Masse von Katalysatoren insbesondere zur katalytischen Oxidation organischer Verbindungen in der Gasphase Verwendung finden, die dadurch erhältlichen Multimetalloxidmassen und ein Verfahren zur Herstellung von Acrylsäure unter Verwendung von Katalysatoren, die diese Multimetalloxidmassen als aktive Masse enthalten.

Multimetalloxidmassen, die abgegrenzte Bereiche einer Promotorphase enthalten, deren chemische Zusammensetzung von der ihrer Umgebung verschieden ist, sind bekannt. Zu ihrer Herstellung wird eine oder mehrere Promotorphasen in feinteiliger Form getrennt vorgebildet und mit Quellen der elementaren Konstituenten einer Wirtsphase gemischt. Das Gemisch wird dann, gegebenenfalls nach Trocknung, calziniert. So beschreiben die DE 198 15 281 und die EP 0 756 894, dass (eine) getrennt vorgebildete Promotorphase(n) mit den Quellen der elementaren Konstituenten der Wirtsphase sowohl trocken als auch nass vermischt werden können. Die getrennt vorgebildete Promotorphase kann dazu einerseits mit feinteiligen Ausgangsverbindungen der wirtsphase im gewünschten Mengenverhältnis in einem Mischer, Kneter oder in einer Mühle trocken vermischt werden können. Andererseits soll die getrennt vorgebildete Promotorphase in eine wässrige Lösung und/oder Suspension von Ausgangsverbindungen der Wirtsphase eingerührt und diese anschließend sprühgetrocknet werden können.

Es hat sich nun gezeigt, dass die bekannten Herstellungsverfahren insbesondere bei der technischen Herstellung großer Katalysatormengen unbefriedigend sind. So wird bei dem trockenen Vermischen der vorgebildeten Promotorphase mit den Ausgangsverbindungen der Wirtsphase nur eine unzureichende mechanische Anbindung der Phasen untereinander erreicht. Andererseits ist beim Einrühren der getrennt vorgebildeten Promotorphase in eine wässrige Lösung und/oder Suspension der elementaren Konstituenten der Wirtsphase aufgrund der hohen Dichte der vorgebildeten Promotorphase nur schwierig eine gleichmäßige Verteilung in der wirtsphase zu erzielen. Außerdem neigen die Kristallite der vorgebildeten Promotorphase beim Einrühren in eine wässrige Lösung und/oder Suspension insbesondere beim längeren Stehenlassen, wie es bei der Verarbeitung großer Mengen unvermeidlich ist, dazu, angelöst zu werden, was die Ausbildung einer scharfen Phasengrenze beeinträchtigt und zu einer verschlechterten Katalysatorleistung führt.

Der vorliegenden Erfindung liegt die Aufgabe zugrunde, ein Verfahren zur Herstellung von Multimetalloxidmassen mit einer Wirtsphase und wenigstens einer darin dispergierten, davon verschiedenen Phase anzugeben, das die genannten Nachteile nicht aufweist. Der Erfindung liegt ferner die Aufgabe zugrunde, ein Verfahren zur Herstellung von Multimetalloxidmassen anzugeben, die beim Einsatz als Katalysatoren der oxidation von Acrolein eine verbesserte Selektivität bezüglich der Bildung von Acrylsäure aufweisen.

Erfindungsgemäß wird diese Aufgabe gelöst durch ein Verfahren zur Herstellung von Multimetalloxidmassen der allgemeinen Formel I

$$[A]_p[B]_q[C]_r \qquad (I),$$

worin A für eine Phase der Zusammensetzung

$$Mo_{12}V_a X^1{}_b X^2{}_c X^3{}_d X^4{}_e X^5{}_f X^6{}_g O_x,$$

B für eine Phase der Zusammensetzung

$$X^7{}_1 Cu_h H_i O_y$$

und C für eine Phase der Zusammensetzung

$$X^8{}_1 Sb_j H_k O_z$$

steht,
wobei die variablen folgende Bedeutung haben:

$X^1$:     W, Nb und/oder Cr,
$X^2$:     Cu, Ni, Co und/oder Fe,

| | |
|---|---|
| $X^3$: | Sb, |
| $X^4$: | Na und/oder K, |
| $X^5$: | Ca, Sr und/oder Ba, |
| $X^6$: | Si, Al und/oder Ti, |
| $X^7$: | Mo und/oder W, |
| $X^8$: | Cu und/oder Zn, bevorzugt Cu, |
| a: | 2 bis 6, |
| b: | 0,5 bis 2,5, |
| c: | 0 bis 4, |
| d: | 0 bis 3, |
| e: | 0 bis 0,3, |
| f: | 0 bis 2, |
| g: | 0 bis 20, |
| h: | 0,5 bis 2, bevorzugt 0,75 bis 1,5, |
| i: | 0 bis 1, |
| j: | 0,2 bis 20, bevorzugt 0,2 bis 5, |
| k: | 0 bis 20, bevorzugt 0 bis 12, |
| x,y,z: | Zahlen, die so gewählt sind, dass in jeder Phase Ladungsneutralität herrscht, und |
| p,q: | positive Zahlen, |
| r: | 0 oder eine positive Zahl, |

wobei das Verhältnis p/(q+r) = 20:1 bis 1:20, vorzugsweise 5:1 bis 1:14 und, für den Fall, dass r für eine positive Zahl steht, das Verhältnis q/r = 20:1 bis 1:20, vorzugsweise 4:1 bis 1:4, beträgt, bei dem man

i) die Phase B und gegebenenfalls C in feinteiliger Form vorbildet,

ii) eine plastisch verformbare Vorläufermasse für die Phase A herstellt,

iii) die vorgebildete Phase B und gegebenenfalls C in der Vorläufermasse für die Phase A dispergiert und die erhaltene Masse trocknet und calziniert.

Der vorliegend verwendete Begriff "Phase" bezeichnet dreidimensional ausgedehnte Bereiche, deren chemische Zusammensetzung von der ihrer Umgebung verschieden ist. Die Phasen sind nicht notwendigerweise röntgenographisch homogen. In der Regel bildet die Phase A eine kontinuierliche Phase, in der Partikel der Phase B und gegebenenfalls C dispergiert sind.

Die feinteiligen Phasen B und gegebenenfalls C bestehen mit Vorteil aus Partikeln, deren Größtdurchmesser, d. h. die längste durch den Schwerpunkt der Partikel gehende Verbindungsstrecke zweier auf der Oberfläche der Partikel befindlicher Punkte, bis zu 300 μm, vorzugsweise 0,1 bis 200 μm, besonders bevorzugt 0,5 bis 50 μm und ganz besonders bevorzugt 1 bis 30 μm beträgt. Geeignet sind aber auch Partikel mit einem Größtdurchmesser von 10 bis 80 μm oder 75 bis 125 μm.

Prinzipiell können die Phasen A, B und C in den erfindungsgemäß hergestellten Multimetalloxidmassen amorph und/oder kristallin vorliegen. Günstig ist es, wenn die Phase B aus Kristalliten von Oxometallaten besteht oder solche Oxometallatkristallite enthält, die das Röntgenbeugungsmuster und damit den Kristallstrukturtyp wenigstens eines der nachfolgenden Kupfermolybdate aufweisen. In Klammern ist die Fundstelle für den zugehörigen Röntgenbeugungsfingerabdruck angegeben.

$Cu_4Mo_6O_{20}$ [A. Moini et al., Inorg. Chem. 25 (21) (1986) 3782 - 3785],

$Cu_4Mo_5O_{17}$ [Karteikarte 39-181 der JCPDS-ICDD Kartei (1991)],

$\alpha$-$CuMoO_4$ [Karteikarte 22-242 der JCPDS-ICDD Kartei (1991)],

$Cu_6Mo_5O_{18}$ [Karteikarte 40-865 der JCPCS-ICDD Kartei (1991)],

$Cu_{4-x}Mo_3O_{12}$ mit x = 0 bis 0,25 [Karteikarte 24-56 und 26-547 der JCPCS-ICDD Kartei (1991)],

$Cu_6Mo_4O_{15}$ [Karteikarte 35-17 der JCPDS-ICDD Kartei (1991)],

$Cu_3(MoO_4)_2(OH)_2$ [Karteikarte 36-405 der JCPDS-ICDD Kartei (1991)],

$Cu_3Mo_2O_9$ [Karteikarte 24-55 und 34-637 der JCPDS-ICDD Kartei (1991)],

$Cu_2MoO_5$ [Karteikarte 22-607 der JCPDS-ICDD Kartei (1991)].

Vorzugsweise enthält die Phase B Oxometallate, die das Röntgenbeugungsmuster und damit den Kristallstrukturtyp des nachfolgenden Kupfermolybdats aufweisen:

$CuMoO_4$-III mit Wolframit-Struktur gemäß Russian Journal of Inorganic Chemistry 36 (7) (1991) 927 - 928, Tabelle 1.
Unter diesen sind diejenigen mit der nachfolgenden Stöchiometrie II

$$CuMO_AW_BO_y \qquad (IV),$$

mit

| | |
|---|---|
| 1/(A+B): | 0,7 bis 1,3, vorzugsweise 0,85 bis 1,15, besonders bevorzugt 0,95 bis 1,05 und ganz besonders bevorzugt 1, |
| A, B: | 0 bis 1 und |
| y | eine Zahl, die durch die Wertigkeit und Häufigkeit der von Sauerstoff verschiedenen Elemente bestimmt wird, |

bevorzugt.

Dieser Strukturtyp wird als HT-Kupfermolybdat-Struktur bezeichnet und ist durch ein Röntgenbeugungsmuster (Fingerabdruck) gekennzeichnet, dessen charakteristischste und intensivste Beugungslinien, angegeben als Netzebenenabstände d [Ä], wie folgt sind:

$6,79 \pm 0,3$
$3,56 \pm 0,3$
$3,54 \pm 0,3$
$3,40 \pm 0,3$
$3,04 \pm 0,3$
$2,96 \pm 0,3$
$2,67 \pm 0,2$
$2,66 \pm 0,2$
$2,56 \pm 0,2$
$2,36 \pm 0,2$
$2,35 \pm 0,2$
$2,27 \pm 0,2$
$2,00 \pm 0,2$
$1,87 \pm 0,2$
$1,70 \pm 0,2$
$1,64 \pm 0,2$
$1,59 \pm 0,2$
$1,57 \pm 0,2$
$1,57 \pm 0,2$
$1,55 \pm 0,2$
$1,51 \pm 0,2$
$1,44 \pm 0,2$

Für den Fall, dass die Phase B ein Gemisch verschiedener Oxometallaten enthält, ist ein Gemisch von Oxometallaten mit Wolframit- und HT-Kupfermolybdat-Struktur bevorzugt. Das Gewichtsverhältnis von Kristalliten mit HT-Kupfermolybdat-Struktur zu Kristalliten mit Wolframit-Struktur kann dabei 0,01 bis 100, 0,1 bis 10, 0,25 bis 4 sowie 0,5 bis 2 betragen.

Die Phase C besteht vorzugsweise aus Kristalliten, die den Trirutil-Strukturtyp des $\alpha$- und/oder $\beta$-Kupferantimonats $CuSb_2O_6$ aufweisen. $\alpha$-$CuSb_2O_6$ kristallisiert in einer tetragonalen Trirutil-Struktur (E.-O. Giere et al., J. Solid State Chem. 131 (1997) 263-274), während $\beta$-$CuSb_2O_6$ eine monoklin verzerrte Trirutil-Struktur aufweist (A. Nakua et al., J. Solid State Chem. 91 (1991) 105-112 oder Vergleichsdiffraktogramm in Karteikarte 17-284 in der JCPDS-ICDD-Kartei 1989). Darüber hinaus werden Phasen C bevorzugt, die die Pyrochlore-Struktur des Minerals Partzite, eines Kupfer-Antimon-Oxid-Hydroxyds mit der variablen Zusammensetzung $Cu_ySb_{2-x}(O,OH,H_2O)_{6-7}$ ($y \leq 2,0$ $\leq x \leq 1$) aufweisen (B. Mason et al., Mineral. Mag. 30 (1953) 100-112 oder Vergleichsdiagramm in Karteikarte 7-303 der JCPDS-ICDD-Kartei 1996).

Des Weiteren kann die Phase C aus Kristalliten bestehen, die die Struktur des Kupferantimonats $Cu_9Sb_4O_{19}$ (S. Shimada et al., Chem. Lett. 1983, 1875-1876 oder S. Shimada et al., Thermochim. Acta 133 (1988) 73-77 oder Vergleichsdiagramm in Karteikarte 45-54 der JCPDS-ICDD-Kartei) und/oder die Struktur von $Cu_4SbO_{4,5}$ (S. Shimada et al., Thermochim. Acta 56 (1982) 73-82 oder S. Shimada et al., Thermochim. Acta 133 (1988) 73-77 oder Vergleichsdiagramm in Karteikarte 36-1106 der JCPDS-ICDD-Kartei) aufweisen.

Selbstverständlich kann die Phase C auch aus Kristalliten bestehen, die ein Gemenge der vorgenannten Strukturen darstellen.

Die Vorabbildung der Phase B kann in einfacher Weise dadurch erfolgen, dass man ein inniges, vorzugsweise feinteiliges Trockengemisch erzeugt, welches geeignete Quellen der elementaren Konstituenten in der gewünschten stöchiometrischen Zusammensetzung enthält, und dieses bei Temperaturen von 200 bis 1000 °C, vorzugsweise 250 bis 800 °C, mehrere Stunden unter Inertgas oder bevorzugt an der Luft calziniert, wobei die Calzinationsdauer einige Minuten bis einige Stunden betragen kann. Dabei kann die Calzinationsatmosphäre zusätzlich Wasserdampf enthalten. Als Quellen für die elementaren Konstituenten der Phase B kommen Oxide und/oder solche Verbindungen in Betracht, die durch Erhitzen, gegebenenfalls in Anwesenheit von Sauerstoff, in Oxide überführbar sind. Neben den Oxiden kommen als solche Ausgangsverbindungen vor allem Halogenide, Nitrate, Formiate, Oxalate, Citrate, Acetate, Carbonate, Amminkomplexsalze, Ammonium-Salze und/oder Hydroxide in Betracht. Verbindungen wie $NH_4OH$, $(NH_4)_2CO_3$, $NH_4NO_3$, $NH_4CHO_2$, $CH_3COOH$, $NH_4CH_3CO_2$ oder Ammoniumoxalat, die spätestens beim späteren Calzinieren zu vollständig gasförmig entweichenden Verbindungen zerfallen und/oder zersetzt werden können, können zusätzlich eingearbeitet werden. Das innige Vermischen der Ausgangsverbindungen kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calzinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Anschließend wird die erhaltene wässrige Masse getrocknet, wobei der Trocknungsprozess vorzugsweise durch Sprühtrocknung der wässrigen Mischung mit Austrittstemperaturen von z. B. 100 bis 150 °C erfolgt. Anschließend wird die getrocknete Masse calziniert.

In einer anderen Herstellvariante der Phase B erfolgt die thermische Behandlung des Gemisches der verwendeten Ausgangsverbindungen in einem Überdruckgefäß (Autoklav) in Gegenwart von überatmosphärischen Druck aufweisendem Wasserdampf bei Temperaturen im Bereich von mehr als 100 bis 600 °C. Der Druckbereich erstreckt sich in typischer Weise auf bis zu 500 atm, vorzugsweise auf bis zu 250 atm. Mit besonderem Vorteil erfolgt diese hydrothermale Behandlung im Temperaturbereich von mehr als 100 bis 374,15 °C (kritische Temperatur der Wassers), in dem Wasserdampf und flüssiges Wasser unter den sich einstellenden Drucken koexistieren.

Die wie beschrieben erhaltenen Multimetalloxidmassen, die Oxometallate eines einzelnen Strukturtyps oder eine Mischung von Oxometallaten verschiedenerer Strukturtypen enthalten können, werden, gegebenenfalls nach Mahlung und/oder Klassierung auf die gewünschte Größe, als feinteilige Phase B eingesetzt.

Die Phase C kann auf die gleiche Weise hergestellt werden, wie oben für die Phase B beschrieben.

Vorzugsweise stellt man ein Trockengemisch der elementaren Konstituenten der Phase C her, das wenigstens einen Teil, vorzugsweise die Gesamtmenge, des Antimons in der Oxidationsstufe +5 enthält und calziniert dieses bei Temperaturen von 200 bis 1200 °C, vorzugsweise von 200 bis 850 °C und besonders bevorzugt von 300 bis 850 °C, unter Inertgas oder bevorzugt an der Luft. Die Calzinationsatmosphäre kann zusätzlich Wasserdampf enthalten. Multimetalloxidmassen, die als Phase C geeignet sind, sind z. B. nach den in der DE-A 24 07 677 ausführlich beschriebenen Herstellweisen erhältlich. Unter diesen ist diejenige Verfahrensweise bevorzugt, bei der man Antimontrioxid oder $Sb_2O_4$ in wässrigem Medium mittels Wasserstoffperoxid in einer Menge, die unterhalb der stöchiometrischen liegt oder ihr gleich ist oder diese übersteigt, bei Temperaturen zwischen 40 und 100 °C zu Antimon(V)oxidhydroxid oxidiert, bereits vor dieser Oxidation, während dieser Oxidation und/oder nach dieser Oxidation wässrige Lösungen und/oder Suspensionen von geeigneten Ausgangsverbindungen der übrigen elementaren Konstituenten der Phase C zusetzt, anschließend das resultierende wässrige Gemisch trocknet (vorzugsweise sprühtrocknet, Eingangstemperatur: 200 bis 600 °C, Ausgangstemperatur: 80 bis 130 °C) und danach das innige Trockengemisch wie beschrieben calziniert.

Bei dem wie eben beschriebenen Verfahren kann man z. B. wässrige Wasserstoffperoxidlösungen in einem $H_2O_2$-Gehalt von 5 bis 33 Gew.-% verwenden. Eine nachträgliche Zugabe von geeigneten Ausgangsverbindungen der übrigen elementaren Konstitutenten der Phase C ist vor allem dann zu empfehlen, wenn diese das Wasserstoffperoxid katalytisch zu zersetzen vermögen. Selbstverständlich könnte man aber auch das Antimon(V)oxidhydroxid aus dem wässrigen Medium isolieren und z. B. mit geeigneten feinteiligen Ausgangsverbindungen der übrigen elementaren Konstituenten der Phase C sowie gegebenenfalls weiteren Sb-Ausgangsverbindungen innig vermischen und anschließend dieses innige Gemisch wie beschrieben calzinieren.

Wesentlich ist, dass es sich bei den Elementquellen der Phase C entweder um Oxide oder um solche Verbindungen handelt, die durch Erhitzen, gegebenenfalls in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate und/oder Hydroxide in Betracht. Verbindungen wie $NH_4OH$, $(NH_4)_2CO_3$, $NH_4NO_3$, $NH_4CHO_2$, $CH_3COOH$, $NH_4CH_3CO_2$ oder Ammoniumoxalat, die spätestens beim späteren Calzinieren zu gasförmig entweichenden Verbindungen zerfallen und/oder zersetzt werden können, können zusätzlich eingearbeitet werden.

Generell kann auch zur Herstellung der Phase C das innige vermischen der Ausgangsverbindungen in trockener oder in nasser Form erfolgen. Erfolgt es in trockener Form, werden die Ausgangsverbindungen zweckmäßigerweise als feinteilige Pulver eingesetzt. Vorzugsweise erfolgt das innige Vermischen jedoch in nasser Form. Üblicherweise werden die Ausgangsverbindungen dabei in Form einer wässrigen Lösung und/oder Suspension miteinander vermischt. Nach Abschluss des Mischvorgangs wird die fluide Masse getrocknet und nach Trocknung calziniert. Vorzugsweise erfolgt auch hier die Trocknung durch Sprühtrocknung. Nach erfolgter Calzinierung können die erhaltenen Oxometallate, z. B. durch Nass- oder Trockenmahlen, z. B. in der Kugelmühle oder durch Strahlmahlen, zerkleinert werden. Aus dem dabei erhältlichen, in der Regel aus im Wesentlichen kugelförmigen Partikeln bestehenden, Pulver kann die Kornklasse mit einem für die Phase C gewünschten Größtdurchmesserbereich abgetrennt werden. Eine bevorzugte Herstellweise einer Phase C der allgemeinen Formel $(Cu,Zn)_1Sb_hH_iO_y$ besteht darin, Antimontrioxid und/oder $Sb_2O_4$ in wässrigem Medium mittels Wasserstoffperoxid zunächst in eine, vorzugsweise feinteilige, Sb(V)-Verbindung, z. B. Sb(V)-Oxidhydroxidhydrat, überzuführen, die dabei resultierende wässrige Suspension mit einer ammoniakalischen wässrigen Lösung von Zn- und/oder Cu-Carbonat (das z. B. die Zusammensetzung $Cu_2(OH)_2CO_3$ aufweisen kann) zu versetzen, die resultierende wässrige Mischung zu trocknen, z. B. sprühzutrocknen, und das erhaltene Pulver, gegebenenfalls nach anschließendem Verkneten mit Wasser und darauffolgendem Verstrangen und Trocknen, wie beschrieben zu calzinieren.

Die plastisch verformbare Vorläufermasse für die Phase A enthält Quellen der elementaren Konstituenten der Phase A. Als geeignete Quellen kommen ebenfalls Oxide und/oder solche Verbindungen, die durch Erhitzen, gegebenenfalls in Anwesenheit von Sauerstoff, in Oxide überführbar sind, in Betracht. Neben den oxiden kommen als solche Ausgangsverbindungen vor allem Halogenide, Nitrate, Formiate, Oxalate, Citrate, Acetate, Carbonate und/oder Hydroxide in Betracht. Verbindungen wie $NH_4OH$, $(NH_4)_2CO_3$, $NH_4HCO_3$, $NH_4NO_3$, $NH_4CHO_2$ $CH_3COOH$ oder $NH_4CH_3CO_2$, die spätestens beim späteren Calzinieren zu vollständig gasförmig entweichenden Verbindungen zerfallen und/oder zersetzt werden können, können zusätzlich eingearbeitet werden. Besonders geeignete Ausgangsverbindungen des Mo, V, W und Nb sind auch deren Oxoverbindungen, d. h. Molybdate, Vanadate, Wolframate und Niobate, bzw. die davon abgeleiteten Säuren und deren Salze, insbesondere die entsprechenden Ammoniumverbindungen, wie Ammoniummolybdat, Ammoniumvanadat, Ammoniumwolframat.

"Plastisch verformbar" bzw. "pastenförmig" bezeichnet eine Konsistenz, die kohärent ist (und nicht, wie ein Pulver aus diskreten Teilchen besteht) und erst unter Einwirkung einer bestimmten Kraft verformbar ist (und nicht, wie eine Lösung oder Suspension, bereitwillig die Gestalt eines Gefäßes annimmt). Die Fließgrenze der plastisch verformbaren Vorläufermasse, d. h. die kleinste Schubspannung, gemessen in Pascal [Pa], oberhalb der sie sich rheologisch wie eine Flüssigkeit verhält, liegt vorzugsweise im Bereich von etwa 1 bis 300 KPa, bevorzugt 10 bis 100 KPa.

Zur Herstellung der plastisch verformbaren Vorläufermasse für die Phase A kann man einer wässrigen Lösung oder Suspension der elementaren Konstituenten der Phase A kontrolliert Wasser entziehen, bis ein pastenförmiger Rückstand erhalten wird. In den meisten Fällen ist es jedoch zweckmäßiger, ein Trockengemisch, welches die Quellen der elementaren Konstituenten der Phase A in der gewünschten stöchiometrischen Zusammensetzung enthält, mit einer geringen Menge eines Lösungsmittels, z. B. 5 bis 100 Gewichtsteilen, vorzugsweise 10 bis 30 Gewichtsteilen, Lösungsmittel pro 100 Gewichtsteile Trockengemisch, zu plastifizieren. Zur Plastifizierung sind alle dem Fachmann bekannten Vorrichtungen zum Homogenisieren hochviskoser Massen, wie insbesondere Kneter, die chargenweise oder kontinuierlich arbeiten können, geeignet. Alternativ kann man auch schnelllaufende Intensivmischer wie Pflugscharmischer, Wendelmischer oder Schräglagemischer verwenden, die gegebenenfalls mit hochtourig laufenden Messerelementen ausgerüstet sind. Man kann auch einen Eirich-Intensiv-Gegenstrommischer oder auch Koller-Mischer einsetzen. Schließlich kann man auch Ringtrogmischer nehmen, wenn man sie mit Kollergängen und hochtourig laufenden Wirbler ausrüstet.

Die Bildung des Trockengemisches kann durch trockenes Vermischen der Ausgangsverbindungen, vorzugsweise in Form feinteiliger Pulver, erfolgen. Vorzugsweise wird das Trockengemisch jedoch dadurch erhalten, dass man die Ausgangsverbindungen unter Bildung einer wässrigen Lösung und/oder Suspension miteinander vermischt. Die Lösung und/oder Suspension wird anschließend getrocknet, vorzugsweise durch Sprühtrocknung, wobei Austrittstemperaturen von 100 bis 150 °C geeignet sind.

Als Lösungsmittel sind alle Lösungsmittel geeignet, die beim Calzinieren bei Temperaturen bis etwa 360 °C weitgehend rückstandsfrei verdampfen oder sich weitgehend rückstandsfrei zersetzen. Das Lösungsmittel wird zweckmäßigerweise so gewählt, dass es das Trockengemisch gut benetzt. Geeignete Lösungsmittel sind beispielsweise Wasser, Carbonsäuren, die verzweigt oder geradkettig, gesättigt oder ungesättigt sein können, wie z. B. Ameisensäure, Essigsäure, primäre oder sekundäre $C_1$-$C_8$-Alkohole, wie Methanol, Ethanol, 2-Propanol, Aldehyde, Ketone, wie Aceton, und Gemische davon. Die beim Trocknen und/oder Calzinieren entweichenden Verdampfungs- und/oder Zersetzungsgase des Lösungsmittels tragen zu einer günstigen Porenstruktur der Multimetalloxidmassen bei.

Als Kneter können z. B. kontinuierliche Schneckenkneter oder Trogkneter verwendet werden. Kontinuierliche Schneckenkneter weisen eine oder mehrere achsparallele, in einem zylindrischen Gehäuse angeordnete Schnecken auf, die auf einer Welle Knet- und Transportelemente aufweisen, die das an einem Ende des Kneters aufgegebene Material zum Austrittsende des Kneters befördern und gleichzeitig plastifizieren und homogenisieren. Besonders bewährt haben sich Trogkneter mit wenigstens zwei horizontal gelagerten Rotoren, z. B. ein sogenannter doppelschaufeliger Trogkneter mit zwei gegenläufig rotierbaren Knetschaufeln in einem Doppelmuldentrog. Die Rotoren können unterschiedliche Formen, wie Sigma-, Mastikator-, Nabenform usw., aufweisen.

In der plastisch verformbaren Vorläufermasse für die Phase A dispergiert man die vorgebildete Phase B und gegebenenfalls C. Zur Dispergierung bzw. Feinverteilung der vorgebildeten Phase B und gegebenenfalls C in der plastisch verformbaren Vorläufermasse für die Phase A sind mit Vorteil die oben genannten Vorrichtungen, wie Kneter, geeignet. Wird, wie in einer bevorzugten Ausführungsform der Erfindung, die plastisch verformbare Vorläufermasse für die Phase A durch Plastifizieren eines Trockengemisches mit einer geringen Menge Lösungsmittel in einer hierzu geeigneten Vorrichtung erhalten, so finden das Plastifizieren und das anschließende Dispergieren der vorgebildeten Phase B und gegebenenfalls C mit Vorteil in der gleichen Vorrichtung statt.

Es ist günstig, wenn das Einarbeiten der vorgebildeten Phase B und gegebenenfalls C in die plastisch verformbare Vorläufermasse für die Phase A bei Temperaturen von weniger als 90 °C, vorzugsweise weniger als 80 °C und besonders bevorzugt weniger als 60 °C erfolgt. In der Regel wird die Einarbeitungstemperatur mehr als 0 °C und meist 20 bis 45 °C betragen.

Weiterhin ist es vorteilhaft, wenn die Zeitspanne vom Dispergieren der vorgebildeten Phase B und gegebenenfalls C in der Vorläufermasse für die Phase A bis zur Trocknung der Masse weniger als 24 Stunden, vorzugsweise weniger als 12 Stunden, insbesondere weniger als 6 Stunden, beträgt.

Die nach dem Dispergieren der vorgebildeten Phase B und gegebenenfalls C in der Vorläufermasse für die Phase A erhaltene Masse kann geeigneterweise zu Formkörpern geformt werden. Hierzu ist die Anwendung eines Extruders besonders geeignet. Das Extrudat kann vorteilhaft Strangform mit einem Durchmesser von z. B. 3 bis 20 mm und einer Länge von z. B. 0,5 bis 4 cm aufweisen.

Die gegebenenfalls geformten Vorläufermassen werden vor der Calzination getrocknet, in der Regel bei 50 bis 180 °C, z. B. etwa 120 °C .

Die Calzination der Vorläufermassen zu den eigentlichen katalytisch aktiven Multimetalloxidmassen erfolgt in der Regel, unabhängig ob vor oder nach erfolgter Formgebung, bei Temperaturen von 250 bis 600 °C, bevorzugt bei Temperaturen von 300 bis 450 °C. Die Calzination kann unter Inertgas, z. B. Stickstoff, einem Gemisch aus Inertgas und Sauerstoff, z. B. Luft, reduzierend wirkenden Gasen, wie Kohlenwasserstoffen, z. B. Methan, Aldehyden, z. B. Acrolein oder Ammoniak, aber auch unter einem Gemisch aus $O_2$ und reduzierend wirkenden Gasen erfolgen, wie es beispielsweise in der DE-A 4335973 beschrieben wird. Bei einer Calzination unter reduzierenden Bedingungen ist allerdings zu beachten, dass die metallischen Konstituenten nicht bis zum Element reduziert werden. zweckmäßigerweise wird die Calzination deshalb unter einer oxidierenden Atmosphäre durchgeführt. Die Calzinationsdauer erstreckt sich in der Regel über einige Stunden und nimmt in üblicher Weise mit zunehmender Calzinierungstemperatur ab. Zur Durchführung der Calzination sind verschiedene mögliche Ofentypen, wie Hordenöfen, Drehrohröfen, Bandcalzinierer, Wirbelbettöfen oder Schachtöfen, geeignet.

Zur Verwendung als Katalysator werden die Multimetalloxidmassen der Formel I in der Regel zu einer gewünschten Katalysatorgeometrie geformt, vorzugsweise durch Aufbringen auf vorgeformte inerte Katalysatorträger, wobei das Aufbringen vor oder nach der abschließenden Calzination erfolgen kann. In der Regel wird die relevante Masse vor der Trägerbeschichtung calziniert. Die Beschichtung der Trägerkörper zur Herstellung der Schalenkatalysatoren wird in der Regel in einem geeigneten drehbaren Behälter ausgeführt, wie er z. B. aus der DE-A 29 09 671 oder aus der EP-A 293 859 bekannt ist. Zweckmäßigerweise kann zur Beschichtung der Trägerkörper die aufzubringende Pulvermasse befeuchtet und nach dem Aufbringen, z. B. mittels heißer Luft, wieder getrocknet werden. Die Schichtdicke der auf den Trägerkörper aufgebrachten Pulvermasse wird zweckmäßigerweise im Bereich 50 bis 500 µm, bevorzugt im Bereich 150 bis 250 µm liegend, gewählt.

Die Pulvermasse kann auch vorteilhaft aus einer Suspension auf die Trägerkörper aufgebracht werden, z. B. durch Aufsprühen der Dispersion auf die bewegten Trägerkörper unter gleichzeitigem Überleiten eines indifferenten Gases wie in der EP-A 15569 beschrieben.

Als Trägermaterialien können dabei übliche poröse oder unporöse Aluminiumoxide, Siliciumdioxid, Thoriumdioxid, Zirkondioxid, Siliciumcarbid oder Silicate, wie Magnesium- oder Aluminiumsilikat, verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder, bevorzugt werden. Von besonderem Vorteil ist die Verwendung von im Wesentlichen unporösen, oberflächenrauhen Ringen aus Steatit, deren Außendurchmesser 4 bis 10 mm beträgt (siehe DE-A 4442346).

Andererseits kann die erfindungsgemäß erhältliche Multimetalloxidmasse auch zur Herstellung von Vollkatalysatoren verwendet werden. Hierzu wird die Vorläufermasse vor oder nach der Calzination zur gewünschten Katalysatorgeometrie verdichtet (z. B. durch Tablettieren, Extrudieren oder Strangpressen), wobei gegebenenfalls die an sich üblichen Hilfsmittel, wie z. B. Graphit oder Stearinsäure, als Gleitmittel und/oder Formhilfsmittel und Verstärkungsmittel, wie Mikrofasern aus Glas, Asbest, Siliciumcarbid oder Kaliumtitanat, zugesetzt werden können. Bevorzugte Vollkatalysatorgeometrien sind Hohlzylinder mit einem Außendurchmesser und einer Länge von 2 bis 10 mm und einer Wandstärke von 1 bis 3 mm.

Die erfindungsgemäß erhältlichen Multimetalloxidmassen eignen sich insbesondere als Katalysatoren mit erhöhter Selektivität (bei vorgegebenem Umsatz) für die gasphasenkatalytische Oxidation von Acrolein zu Acrylsäure. Normalerweise wird bei dem Verfahren Acrolein eingesetzt, das durch die katalytische Gasphasenoxidation von Propen erzeugt wurde. In der Regel werden die Acrolein enthaltenden Reaktionsgase dieser Propenoxidation ohne Zwischenreinigung eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt (z. B. in Form von Luft), eingesetzt. Geeignete Verdünnungsgase sind z. B. $N_2$, $CO_2$, Kohlenwasserstoff, rückgeführte Reaktionsabgase und/oder Wasserdampf. In der Regel wird bei der Acrolein-Oxidation ein Acrolein:Sauerstoff:Wasserdampf:Inertgas-Volumenverhältnis von 1:(1 bis 3):(0 bis 20):(3 bis 30) vorzugsweise von 1:(1 bis 3):(0,5 bis 10):(7 bis 18) eingestellt. Der Reaktionsdruck beträgt im Allgemeinen 1 bis 3 bar und die Gesamtraumbelastung beträgt vorzugsweise 1000 bis 4500 N1/(1·h). Typische Vielrohr-Festbettreaktoren sind z. B. in den Schriften DE-A 28 30 765, DE-A 22 01 528 oder US-A 3 147 084 beschrieben. Die Reaktionstemperatur wird üblicherweise so gewählt, dass der Acrolein-Umsatz bei einfachem Durchgang oberhalb von 90 %, vorzugsweise oberhalb von 98 %, liegt. Im Normalfall sind diesbezüglich Reaktionstemperaturen von 230 bis 330 °C erforderlich.

Neben der gasphasenkatalytischen Oxidation von Acrolein zu Acrylsäure vermögen die erfindungsgemäßen Verfahrensprodukte aber auch die gasphasenkatalytische Oxidation anderer organischer Verbindungen wie insbesondere anderer, vorzugsweise 3 bis 6 C-Atome aufweisender Alkane, Alkanole, Alkanale, Alkene und Alkenole (z. B. Propylen, Methacrolein, tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung olefinischer Verbindungen.

Im Übrigen sind in dieser Schrift Umsatz, Selektivität und Verweilzeit, falls nichts anderes erwähnt wird, wie folgt definiert:

$$\text{Umsatz U an Acrolein \%} = \frac{\text{Molzahl umgesetztes Acrolein}}{\text{Molzahl eingesetztes Acrolein}} \times 100$$

$$\text{Selektivität S der Acrylsäurebildung} = \frac{\text{Molzahl umgestzt su Acryläure}}{\text{Molzahl umgesetztes Acrolein}} \times 100$$

$$\text{Verweilzeit (sec)} = \frac{\text{mit Katalysator gefülltes Leervolumen des Reaktors (l)}}{\text{durchgesetzte Synthesegasmenge (Nl/h)}} \times 3600$$

Herstellungsbeispiel 1

a) Vorabherstellung der Phase B der Stöchiometrie $Cu_{1,0}Mo_{0,5}W_{0,5}O_4$

In einem ersten Rührkessel wurden bei etwa 25 °C unter Rühren 620 1 Wasser vorgelegt. Anschließend gab man 27,4 kg $(NH_4)_6Mo_7O_{24}*4H_2O$ dazu. Nach Erwärmen gab man 40,4 kg $(NH_4)_{10}W_{12}O_{41}*7H_2O$ hinzu und erhitzte das Gemisch unter weiterem Rühren auf 90 °C. Es wurde eine klare gelborange Lösung erhalten (Lösung 1).

Parallel zur Herstellung der Lösung 1 wurden in einem zweiten Rührkessel bei etwa 25 °C unter Rühren 373 l Wasser vorgelegt. Anschließend wurden 61 l 25 gew.-%ige wässrige $NH_3$-Lösung eingerührt. Zu der ammoniakalischen Lösung gab man 61,9 kg Kupfer(II)acetat und rührte das erhaltene Gemisch bis zum Erhalt einer klaren, dunkelblauen Lösung ohne Bodensatz (Lösung 2).

Lösung 2 wurde aus dem zweiten Rührkessel in die Lösung 1 überführt. Anschließend wurde die erhaltene türkisfarbene Maische sprühgetrocknet, wobei die Gaseintrittstemperatur am Sprühturm 250 °C, die Gasaustrittstemperatur 140 °C betrug. Die Sprühtrocknung erfolgte im Gleichstrom.

75 kg des erzeugten Sprühpulvers wurden in einen Kneter dosiert und unter Zugabe von 15 l Wasser geknetet. Danach wurde das Knetgut in einen Extruder entleert und mittels des Extruders zu Strängen (Länge 1-2 cm; Durchmesser 6 mm) geformt.

Auf einem Bandtrockner wurden die Stränge bei einer Temperatur von 120 °C getrocknet.

Der Katalysatorvorläufer wurde im Drehrohr in kontinuierlicher Fahrweise bei einer Temperatur von 340 °C und einer Verweilzeit von wenigstens 1 h im Luftstrom calziniert. Anschließend wurde der Vorläufer nochmals bei 790 °C calziniert.

Anschließend wurden die Stränge in einer Mühle (Biplexmühle BQ 500) auf einen mittleren Partikeldurchmesser von 3 bis 5 $\mu$m gemahlen.

Der resultierende Split wies eine BET-Oberfläche $\leq$ 1 m$^2$/g auf. Mittels Röntgenbeugung wurden folgende Phasen festgestellt:

1. Cu MoO$_4$-II mit Wolframitstruktur,
2. HT-Kupfermolybdat.

b) Vorabherstellung der vorläufermasse für die Phase A der Stöchiometrie Mo$_{10,4}$V$_3$W$_{1,2}$O$_x$

In einem Rührkessel wurden bei etwa 25 °C unter Rühren (70 Upm) 900 l Wasser vorgelegt. Anschließend gab man 122,4 kg (NH$_4$)$_6$Mo$_7$O$_{24}$*H$_2$O dazu und erhitzte das Gemisch unter Rühren auf 90 °C. Anschließend wurden 22,2 kg NH$_4$VO$_3$ (Ammoniummetavanadat) zugegeben. Dann wurden 20,9 kg (NH$_4$)$_{10}$W$_{12}$O$_{41}$*7H$_2$O zugegeben; durch 60-minütiges Rühren bei 90 °C erhielt man eine klare orangefarbene Lösung. Ihr pH-Wert betrug 6,2 $\pm$ 0,3. Durch Zugabe von Essigsäure wurde der pH-Wert zunächst auf 5,0 $\pm$ 0,3 gesenkt und anschließend durch Einrühren von 25 gew.-%iger wässriger NH$_3$-Lösung wieder auf 6,2 $\pm$ 0,3 erhöht. Im Ergebnis wurde eine klare, orangefarbene Lösung ohne Bodensatz erhalten. Diese wurde anschließend sprühgetrocknet, wobei die Gaseintrittstemperatur am Sprühturm 240 °C, die Gasaustrittstemperatur 100 °C betrug. Das erhaltene Sprühpulver war hellgelb.

c) Herstellung der Multimetalloxidaktivmasse

75 kg des unter b) erhaltenen Sprühpulvers wurden in einem Trogkneter mit zwei Z-förmigen, horizontal gelagerten Knetarmen vorgelegt. Dann wurden 8,6 l Essigsäure zugegeben und anschließend durch Zugabe der erforderlichen Menge Wasser eine knetbare Konsistenz eingestellt. Anschließend wurden 12,9 kg der vorab hergestellten Phase B zugegeben und bis zur Homogenität geknetet. Danach wurde das Knetgut in einen Extruder entleert und mittels des Extruders zu Strängen (Länge 1-2 cm; Durchmesser 6 mm) geformt. Auf einem Bandtrockner wurden die Stränge bei einer Temperatur von 120 °C getrocknet.

300 kg der so erzeugten Formlinge wurden auf einen Hordenwagen geladen, der mit 10 Blechen von jeweils 1 m Tiefe und 50 cm Breite bestückt war. Die Bleche waren in zwei Reihen nebeneinander und äquidistant übereinander angeordnet. Die Bleche bestanden aus einem Lochblech mit einem Lochdurchmesser von 3 mm. Der Hordenwagen wurde in einen Hordenofen (Innenmaße: Höhe x Breite x Tiefe: 1,30 m x 1,18 m x 1,10 m) eingeschoben, der im Umluftbetrieb mit einer Umluftmenge von etwa 2500 m$^3$/h betrieben wurde. Das Prozessgas wurde elektrisch erwärmt; die Temperaturregelung erfolgt über ein Thermoelement im Gasstrom. Die Produkttemperatur wurde durch 20 Thermoelemente überwacht, die etwa in der Mitte der Bleche in der Mitte der Produktschüttung angebracht waren. Die Formlinge wurden so in einer Gasatmosphäre von 1,5 Vol-% O$_2$, 7 Vol-% NH$_3$, Rest N$_2$ folgendermaßen calziniert:

Es wurde mit einer Geschwindigkeit von 5 °C pro min die Temperatur des den Hordenofen durchströmenden Gasgemisches in entsprechenden Heizzonen des Hordenofens von Raumtemperatur auf 325 °C erhöht und 11 h auf dieser Temperatur gehalten. Anschließend wurde der NH$_3$-Gehalt der Gasatmosphäre auf 0 % gesenkt. Danach wurde in entsprechender weise mit einer Geschwindigkeit von 2,5 °C/min auf 400 °C aufgeheizt und diese Temperatur 80 min aufrecht erhalten. Anschließend wurde auf Raumtemperatur abgekühlt.

Die so erhaltenen calzinierten Stränge wurden in einer Mühle (Biplexmühle BQ 500) zu zweiphasigem Multimetalloxidaktivmassensplit eines mittleren Korndurchmessers von 3 bis 5 $\mu$m gemahlen.

d) Katalysatorherstellung

In einer Beschichtungstrommel wurden 70 kg Steatitringe (Außendurchmesser x Höhe x Innendurchmesser = 7 mm x 3 mm x 4 mm) als Katalysatorträger vorgelegt und wie folgt mit Multimetalloxidaktivmassensplit beschichtet:

Mit einer Dosierrinne wurden insgesamt 18,1 kg Multimetalloxidaktivmassensplit in die Beschichtungstrommel gegeben. Parallel dazu wurde ein Glycerin/Wasser-Gemisch (Gewichtsverhältnis Glycerin:wasser = 3:1) in einer Gesamtmenge von 3,5 l als Haftflüssigkeit zudosiert.

Abschließend wurde in der Beschichtungstrommel getrocknet. Der Aktivmassenanteil der so erzeugten Schalenkatalysatoren betrug etwa 20 % ihres Gewichts.

Herstellungsbeispiel 2

Herstellungsbeispiel 1 wurde wiederholt, wobei jedoch in Schritt b) eine Vorläufermasse für die Phase A der Stöchiometrie $Mo_{10,4}V_4W_{1,2}O_x$ hergestellt wurde, indem 29,6 kg Ammoniummetavanadat verwendet wurden.

Herstellungsbeispiel 3

Herstellungsbeispiel 1 wurde wiederholt, wobei jedoch in Schritt c) nur 10,3 kg der vorgebildeten Phase B eingesetzt wurden.

Beispiel 1: Gasphasenkatalytische Festbettoxidation

Zur gasphasenkatalytischen Festbettoxidation von Acrolein zu Acrylsäure wurden die Kontaktrohre eines Rohrbündelreaktors mit dem Katalysator aus Herstellungsbeispiel 1 als Festbett beschickt. Dabei wurde in Strömungsrichtung des Reaktionsgasgemisches zunächst mit einem Gemisch aus unbeschichteten Steatitringen (30 Vol-%) und Ringkatalysatoren (70 Vol-%) beschickt und anschließend ausschließlich mit Ringkatalysatoren.

Die Zusammensetzung des eingeführten Gasgemisches betrug etwa:

5,5 Vol.-% Acrolein,
0,3 Vol.-% Propen,
0,55 Vol.-% CO,
1,0 Vol.-% $CO_2$,
0,3 Vol.-% Acrylsäure,
7,0 Vol.-% $H_2O$,
6,0 Vol.-% Sauerstoff und als Restmenge Stickstoff.

Umsatz U und Selektivität S der Acrylsäurebildung waren wie folgt:

Belastung: 87 Nl Acrolein/$_{lkat}\cdot$h

$U = 99,5\ \%$, $S_{ACS} = 96\ \%$, $T = 260\ °C$

Beispiel 2:

Beispiel 1 wurde wiederholt, wobei jedoch die Belastung 130 Nl Acrolein/$l_{kat}\cdot$h war. Umsatz U und Selektivität S der Acrylsäurebildung waren wie folgt:

$U = 99,5\ \%$, $S_{ACS} = 96\ \%$, $T = 267\ °C$

Beispiel 3

Beispiel 1 wurde wiederholt, wobei jedoch der Katalysator aus Herstellungsbeispiel 2 eingesetzt wurde. Umsatz U und Selektivität S der Acrylsäurebildung waren wie folgt:

$U = 99,5\ \%$, $S_{ACS} = 95,8\ \%$, $T = 262\ °C$

Beispiel 4

Beispiel 3 wurde wiederholt, wobei jedoch die Belastung 130 Nl Acrolein/$l_{kat}\cdot$h war. Umsatz U und Selektivität S der Acrylsäurebildung waren wie folgt:

$U = 99,4\ \%$, $S_{ACS} = 95,9\ \%$, $T = 271\ °C$

Beispiel 5

Beispiel 1 wurde wiederholt, wobei jedoch der Katalysator aus Herstellungsbeispiel 3 verwendet wurde. Umsatz U und Selektivität S der Acrylsäurebildung waren wie folgt:

U = 99,5 %, $S_{ACS}$ = 95,9 %, T = 255 °C

Beispiel 6

Beispiel 5 wurde wiederholt, wobei jedoch die Belastung 130 Nl Acrolein/$l_{kat} \cdot$h war. Umsatz U und Selektivität S der Acrylsäurebildung waren wie folgt:

U = 99,5 %, $S_{ACS}$ = 95,9 %, T = 264 °C

**Patentansprüche**

1. Verfahren zur Herstellung von Multimetalloxidmassen der allgemeinen Formel I

$$[A]_p[B]_q[C]_r \qquad (I),$$

worin A für eine Phase der Zusammensetzung

$$Mo_{12}V_aX^1_bX^2_cX^3_dX^4_{½e}X^5_fX^6_gO_x,$$

B für eine Phase der Zusammensetzung

$$X^7_1Cu_hH_iO_y$$

und C für eine Phase der Zusammensetzung

$$X^8_1Sb_jH_kO_z$$

steht,
wobei die Variablen folgende Bedeutung haben:

$X^1$: W, Nb und/oder Cr,
$X^2$: Cu, Ni, co und/oder Fe,
$X^3$: Sb,
$X^4$: Na und/oder K,
$X^5$: Ca, Sr und/oder Ba,
$X^6$: Si, Al und/oder Ti,
$X^7$: Mo und/oder W,
$X^8$: Cu und/oder Zn,
a: 2 bis 6,
b: 0,5 bis 2,5,
C: 0 bis 4,
d: 0 bis 3,
e: 0 bis 0,3,
f: 0 bis 2,
g: 0 bis 20,
h: 0,5 bis 2,
i: 0 bis 1,

j:        0,2 bis 20,

k:        0 bis 20,

x,y,z:    Zahlen, die so gewählt sind, dass in jeder Phase Ladungsneutralität herrscht, und

p,q:     positive Zahlen,

r:        0 oder eine positive Zahl,

wobei das Verhältnis p/(q+r) = 20:1 bis 1:20 und, für den Fall, dass r für eine positive Zahl steht, das Verhältnis q/r = 20:1 bis 1:20 beträgt, bei dem man

i) die Phase B und gegebenenfalls C in feinteiliger Form vorbildet,

ii) eine plastisch verformbare Vorläufermasse für die Phase A herstellt,

iii) die vorgebildete Phase B und gegebenenfalls C in der Vorläufermasse für die Phase A dispergiert und die Masse trocknet und calziniert.

2. Verfahren nach Anspruch 1, **dadurch gekennzeichnet, dass** man die plastisch verformbare Vorläufermasse für die Phase A erhält, indem man ein Trockengemisch, das Quellen der elementaren Konstitutenten der Phase A enthält, mit 5 bis 100 Gewichtsteilen eines Lösungsmittels pro 100 Gewichtsteile Pulver plastifiziert.

3. Verfahren nach Anspruch 2, **dadurch gekennzeichnet, dass** das Lösungsmittel unter Wasser, Carbonsäuren, primären oder sekundären $C_1$-$C_8$-Alkoholen, Aldehyden, Ketonen und Gemischen davon ausgewählt ist.

4. Verfahren nach Anspruch 2 oder 3, **dadurch gekennzeichnet, dass** man das Trockengemisch in einem Trogkneter mit wenigstens zwei horizontal gelagerten Rotoren plastifiziert und die vorgebildete Phase B und gegebenenfalls c im gleichen Kneter in der vorläufermasse für die Phase A dispergiert.

5. Verfahren nach einem der vorhergehenden Ansprüche, **dadurch gekennzeichnet, dass** das Dispergieren der vorgebildeten Phase B und gegebenenfalls C in der Vorläufermasse für die Phase A bei einer Temperatur von 90 °C oder weniger erfolgt.

6. Verfahren nach einem der vorhergehenden Ansprüche, **dadurch gekennzeichnet, dass** die Zeitspanne vom Dispergieren der vorgebildeten Phase B und gegebenenfalls C in der Vorläufermasse für die Phase A bis zur Trocknung der Masse weniger als 24 Stunden beträgt.

7. Verfahren nach Anspruch 6, **dadurch gekennzeichnet, dass** die » Zeitspanne vom Dispergieren der vorgebildeten Phase B und gegebenenfalls C in der Vorläufermasse für die Phase A bis zur Trocknung der Masse weniger als 12 Stunden beträgt.

8. Multimetalloxidmasse, erhältlich nach einem Verfahren gemäß einem der vorhergehenden Ansprüche.

9. Katalysator, enthaltend als aktive Masse eine Multimetalloxidmasse nach Anspruch 8.

10. Verfahren zur Herstellung von Acrylsäure durch gasphasenkatalytische Oxidation von Acrolein, **dadurch gekennzeichnet, dass** als Katalysator ein Katalysator gemäß Anspruch 9 verwendet wird.

**Claims**

1. A process for preparing multimetal oxide compositions of the formula ₁

$$[A]_p[B]_q[C]_r \qquad (I),$$

where A is a phase having the composition

$$Mo_{12}V_aX^1{}_bX^2{}_cX^3{}_dX^4{}_eX^5{}_fX^6{}_gO_x,$$

B is a phase having the composition

$$X^7{}_1Cu_hH_iO_y$$

and C is a phase having the composition

$$X^8{}_1Sb_jH_kO_z$$

where the variables have the following meanings:

| | |
|---|---|
| $X^1$: | W, Nb and/or Cr, |
| $X^2$: | Cu, Ni, Co, and/or Fe, |
| $X^3$: | Sb, |
| $X^4$: | Na, and/or K, |
| $X^5$: | Ca, Sr and/or Ba, |
| $X^6$: | Si, Al, and/or Ti, |
| $X^7$: | Mo and/or W, |
| $X^8$: | Cu and/or Zn, |
| a: | 2 to 6, |
| b: | 0.5 to 2.5, |
| c: | 0 to 4, |
| d: | 0 to 3, |
| e : | 0 to 0.3, |
| f | 0 to 2, |
| g: | 0 to 20, |
| h: | 0.5 to 2, |
| i: | 0 to 1, |
| j: | 0.2 to 20, |
| k: | 0 to 20, |
| x,y,z: | numbers selected so that each phase is electrically neutral, and |
| p,q: | positive numbers, |
| r: | 0 or a positive number, |

where the ratio p/(q+r) = 20:1 to 1:20 and, when r is a positive number, the ratio q/r = 20:1 to 1:20, which comprises

i) preforming the phase B and optionally C in finely divided form,

ii) preparing a plasticially deformable precursor composition for the phase A, and

iii) dispersing the preformed phase B and optionally C in the precursor composition for the phase A and drying and calcining the composition.

2. A process as claimed in claim 1, wherein the plastically deformable precursor composition for the phase A is obtained by plasticizing a dry mixture comprising sources of the elemental constituents of the phase A by means of from 5 to 100 parts by weight of a solvent per 100 parts by weight of powder.

3. A process as claimed in claim 2, wherein the solvent is selected from among water, carboxylic acids, primary and secondary $C_1$-$C_8$-alcohols, aldehydes, ketones and mixtures thereof.

4. A process as claimed in claim 2 or 3, wherein the dry mixture is plasticized in a trough kneader having at least two horizontally mounted rotors and the preformed phase B and optionally C is/are dispersed in the precursor composition for the phase A in the same kneader.

5. A process as claimed in any of the preceding claims, wherein dispersion of the preformed phase B and optionally C in the precursor composition for the phase A is carried out at 90°C or below.

6. A process as claimed in any of the preceding claims, wherein the time from dispersion of the preformed phase B and optionally C in the precursor composition for the phase A to drying of the composition is less than 24 hours.

7. A process as claimed in claim 6, wherein the time from dispersion of the preformed phase B and optionally C in the precursor composition for the phase A to drying of the composition is less than 12 hours.

8. A multimetal oxide composition obtainable by a process as claimed in any of the preceding claims.

9. A catalyst comprising a multimetal oxide composition as claimed in claim 8 as active composition.

10. A process for preparing acrylic acid by gas-phase catalytic oxidation of acrolein, wherein a catalyst as claimed in claim 9 is used.


**Revendications**

1. Procédé de préparation de masses d'oxydes multimétalliques de la formule générale I

$$[A]_p \, [B]_q \, [Cl]_r \qquad\qquad (I),$$

dans laquelle a représente une phase de composition

$$MO_{12}V_a X^1{}_b \, X^2{}_c X^3{}_d \, X^4{}_e X^5{}_f X^6{}_g O_x,$$

B une phase de composition

$$X^7{}_l Cu_h H_i O_y$$

et C une phase de composition

$$X^8{}_l Sb_j H_k O_z,$$

où les variables ont la signification suivante:

| | |
|---|---|
| $X^1$: | W, Nb et/ou Cr, |
| $X^2$: | Cu, Ni, Co et/ou Fe, |
| $X^3$: | Sb, |
| $X^4$: | Na et/ou K, |
| $X^5$: | Ca, Sr et/ou Ba, |
| $X^6$: | Si, Al et/ou Ti, |
| $X^7$: | Mo et/ou W, |
| $X^8$: | Cu et/ou Zn, |
| a: | 2 à 6, |
| b: | 0,5 à 2,5, |
| c: | 0 à 4, |
| d: | 0 à 3, |
| e: | 0 à 0,3, |
| f: | 0 à 2, |
| g: | 0 à 20, |
| h: | 0,5 à 2, |

i:          0 à 1,
j:          0,2 à 20,
k:          0 à 20,
x, y, z:    des nombres choisis de manière qu'il règne dans chaque phase une neutralité de charge, et
p, q:       des nombres positifs,
l:          0 ou un nombre positif,

le rapport p/(q + r) = 20 : 1 à 1 : 20 et, dans le cas où r représente un nombre positif, le rapport q/r = 20 : 1 à 1 : 20, dans lequel

> i) on prépare la phase B et éventuellement C sous forme finement divisée,
> ii) on prépare une masse progénitrice de la phase A déformable de manière plastique,
> iii) on disperse la phase B et éventuellement C préparée(s) dans la masse progénitrice de la phase A, et l'on sèche et calcine la masse.

2. Procédé selon la revendication 1, **caractérisé en ce que** l'on obtient la masse progénitrice de la phase A, déformable de manière plastique, **en ce que** l'on plastifie un mélange sec contenant des sources des constituants élémentaires de la phase A au moyen de 5 à 100 parties en poids d'un solvant pour 100 parties en poids de poudre.

3. Procédé selon la revendication 2, **caractérisé en ce que** le solvant est choisi parmi de l'eau, des acides carboxyliques, des alcools primaires ou secondaires en $C_1$ à $C_8$, des aldéhydes, des cétones, et leurs mélanges.

4. Procédé selon la revendication 2 ou 3, **caractérisé en ce que** l'on plastifie le mélange sec dans un malaxeur à bac comportant au moins deux rotors placés horizontalement, et que l'on disperse la phase préparée B et éventuellement C, dans le même malaxeur, dans la masse progénitrice de la phase A.

5. Procédé selon l'une quelconque des revendications qui précèdent, **caractérisé en ce que** la dispersion de la phase préparée B et éventuellement C dans la masse progénitrice de la phase A est entreprise à une température de 90°C ou moins.

6. Procédé selon l'une quelconque des revendications qui précèdent, **caractérisé en ce que** la durée de la dispersion de la phase préparée B et éventuellement C dans la masse progénitrice de la phase A jusqu'au séchage de la masse est de moins de 24 heures.

7. Procédé selon la revendication 6, **caractérisé en ce que** la durée de la dispersion de la phase préparée B et éventuellement C dans la masse progénitrice de la phase A jusqu'au séchage de la masse est de moins de 12 heures.

8. Masses d'oxydes multimétalliques, que l'on peut obtenir par un procédé selon l'une quelconque des revendications qui précèdent.

9. Catalyseur contenant comme masse active une masse d'oxyde multimétallique selon la revendication 8.

10. Procédé de préparation d'acide acrylique par oxydation catalytique en phase gazeuse d'acroléine, **caractérisé en ce que** l'on utilise comme catalyseur un catalyseur selon la revendication 9.